# EUROPEAN PATENT SPECIFICATION

(11) **EP 1 113 269 B1**
(45) Date of publication and mention of the grant of the patent: **18.10.2006**
(21) Application number: 00111483.4
(22) Date of filing: 29.05.2000
(51) Int. Cl.: G01N 33/48, G01N 33/68, B01L 3/00, H01J 49/04

(54) **Test tray, kit and methods for screening body fluids of newborns by tandem mass spectrometry**
Testtablett, Kit und Verfahren zum Screening von Körperflüssigkeiten von Neugeborenen durch Tandem-Massenspektrometrie
Cuvette d'essai, trousse de réactifs et méthodes pour criblage des fluides corporels des nouveau-nés par spectrométrie de masse de type tandem

(30) Priority: 29.12.1999 US 474604
(43) Date of publication of application: 04.07.2001
(73) Proprietor: PerkinElmer Life Sciences, Inc., Norton, Ohio 44203-6215 (US)
(72) Inventor: Ostrup, Jan, 20780 Kaarina (FI)
(74) Representative: Becker Kurig Straus

(56) References cited:
- SCHRAAG B ET AL: "Standardization of an enzyme-linked immunosorbent assay for the determination of protein tyrosine kinase activity." ANALYTICAL BIOCHEMISTRY, vol. 211, no. 2, 1993, pages 233-239, XP009007839 ISSN: 0003-2697
- NAYLOR EDWIN W ET AL: "Automated tandem mass spectrometry for mass newborn screening for disorders in fatty acid, organic acid, and amino acid metabolism." JOURNAL OF CHILD NEUROLOGY, vol. 14, no. SUPPL. 1, November 1999 (1999-11), pages S4-S8, XP000980271 ISSN: 0883-0738

## Description

### TECHNICAL FIELD

The present invention is in the field of equipment, kits and methods for testing bodily fluids for newborn screening by tandem mass spectrometry kits.

### BACKGROUND

Newborn infants are screened for metabolic disorders in all developed countries of the world. In addition, newborn screening is beginning to be adopted by many developing countries around the world. Prior to the use of tandem mass spectrometry by some newborn screening programs, the number of inherited disorders screened has been limited in most programs. In the US, typically a program will screen for no more than six disorders.

To increase the number of disorders screened and to screen for fatty acid metabolism disorders, some programs have adopted tandem mass spectrometry for screening newborns. This allows screening for multiple disorders from a single blood spot punch. The tandem mass screening is typically carried out by extracting a sample of the child's blood from the punched blood spot with a solvent, such as methanol, which also contains isotopically labelled standards of known concentration. The amount of material measured in the blood spot extract by tandem mass spectrometry is inferred by observation of the ratio of instrument response of the known standard to the unknown substance undergoing analysis. Usually the internal standards are prepared as a solution and stored over a period of time, until the solution is consumed. Storage of these alcoholic solutions of internal standards can lead to problems in quantitation due to evaporation of the solvent. This evaporation can occur over periods of time even if precautions are taken to avoid it due to the repeated opening of the standard solution container for use. Any carelessness in use or storage of the standard solutions will exacerbate this problem. Evaporation of the solvent will lead to incorrect calculation of the amount of analyte because the concentration of the standards is no longer reliably known.

When the standards are stored as solids, those such as the acylcarnitines are subject to degradation through hydrolysis. This has limited the usefulness of dry standards, since they must be stored under inert gasses and at low temperatures. The solid standards were known to be stable at low temperatures when stored under nitrogen. These conditions do not allow for an efficient manufacture of individual samples of the standards, their economical shipment to customer sites, or the storage of the individual samples at the user site.

Schraag B. et al. discloses in Analytical Biochemistry, Vol. 211, NO. 2, 1993, pages 233-239 a procedure for an enzyme-linked immunosorbent assay for the determination of protein tyrosine kinase (PTK) activity from cytosolic and solubilized membrane fractions from breast cancers. The general PTK substrate is coated to the wells of a microtiter plate. After incubation with PTK sample and ATP the amount of phosphorylated tyrosyl residues is quantitated with phosphotyrosine specific antibodies and a secondary peroxidase-labelled antibody. Tyrosine is used as dried internal standard. Also in this document it is disclosed a method of making a test tray comprising a plurality of test cells comprising placing an aliquot of a liquid containing an internal standard in each of said test cells of said tray and drying said liquid containing the internal standard. It is also described that the wells of a microtiter plate are coated with either BSA-phosphotyrosine or poly(glutamic acid: tyrosine, PGT).

Naylor Edwin W. et al. describes in Journal Of Child Neurology, Vol. 14, NO. Suppl. 1, November 1999 (1999-11), pages S4-S8, that isotope labelled internal standards can be used in automatic tandem mass spectrometry for mass newborn screening for disorders in fatty acid, organic acid and amino acid metabolism.

Accordingly, it is desirable to be able to produce test trays and test kits, and related methods, that provide for more accurate and reliable testing through use of more concentration stable internal standards.

Is also desirable to produce test trays and test kits that offer convenient storage, transport and use of internal standards attendant to testing such as newborn screening. Is also beneficial to be able to provide these advantages while maintaining the stability of these internal standards.

Although described against the backdrop of newborn screening techniques, other advantages of the present invention may become apparent to one of ordinary skill through use of the invention in this field or in other applications.

### SUMMARY OF THE INVENTION

Accordingly, the present invention includes test trays, a method of their manufacture, and kits and analytical methods using them.

The testing tray for conducting a plurality of tests on a biological fluid comprises: (a) a test tray comprising a plurality of cells; and (b) at least two of the cells containing a dried internal standard used in each respective ones of the plurality of tests.

The dried internal standard includes an isotope labelled standard selected from the group consisting of carnitine, acetyl carnitine, propionyl carnitine, butryl carnitine, isovaleryl carnitine, octanoyl carnitine, myristoyl carnitine, palmitoyl carnitine, glycine, alanine, valine, leucine, methionine, phenylalanine, tyrosine, aspartate, glutamate, ornithine, citrulline, arginine, polysaccharides, polysaccharides bound to protein or protein fragments, and mixtures thereof.

It is also preferred that the test tray be provided with a cover piece, such as a polymeric, aseptic covering, and that it be packaged to prevent contamination and to facilitate storage and shipping.

The present invention also includes a method of conducting a plurality of in vitro tests to assay analytes in a bodily fluid sample fluid, the method comprising: (a) providing a sample of bodily fluid; (b) placing aliquots of the bodily fluid sample in a plurality of cells of a test tray, the test tray comprising: (i) a plurality of cells; and (ii) at least two of the cells containing a dried internal standard (as described above) used in each of the tests; so as to produce a respective plurality of test/standard samples, followed by (c) performing an assay test on each of the respective plurality of test/standard samples.

The tests performed on each of the respective plurality of test/standard samples may include at least two tests adapted to assay different analytes from respective plurality of test/standard samples, or may include at least two tests adapted to assay the same analyte from respective test/standard samples (such as for repetitive testing), or a combination thereof.

Typically the plurality of tests will be conducted using tandem mass spectrometry.

The present invention also includes a kit including a test tray for conducting a plurality of tests to assay analytes in a bodily fluid, the kit comprising: (a) a test tray comprising a plurality of cells; (b) at least two of the cells containing said dried internal standard; (c) a solvent for addition to the cells containing a dried internal standard (as described above); and (d) an optional solvent dispenser (such as a micropipette).

The present invention also features a newborn screening method for conducting a plurality of tests on blood from a newborn human to assay at least one analyte therefrom, the method comprising: (a) obtaining a test tray, the test tray comprising: (i) a plurality of cells; and (ii) at least two of the cells containing a dried internal standard (as described above) used in each of the tests performed respectively on each test sample/standard in each cell after being provided with a solvent and blood sample; (b) placing a plurality of sample paper portions each bearing dried sample of blood from a newborn human into respective cells of the test tray; (c) placing aliquots of the blood into respective cells of the test tray so as to produce a respective plurality of test/standard samples; and (d) performing tandem mass spectrometry on each one of the respective plurality of test/standard samples.

The blood samples may be taken from blood spot sample paper that may be delivered to the laboratory from hospitals or clinics. Otherwise the blood sample may be provided in any other form appropriate to the desired test(s) and/or application, such as in the form of hemosylate, stored liquid blood or blood products or freeze dried samples, etc.

The tests performed on each of the respective plurality of test/standard samples might include the same or different tests or combination thereof as described herein. These may be tests for one or more of a wide variety of analytes (such as those measured against known or empirically developed thresholds or ranges) indicative of metabolic disorders, or otherwise indicating the need for further study of the tested individual.

The dried internal standard may be any appropriate isotope labelled standard for the desired test(s). The dried internal standards may be any standards known in the art, and may include those isotopically labelled either singly or in combination by H², C¹³ and N¹⁵, such those mentioned herein.

The method of the present invention also includes a method for conducting a plurality of in vitro tests on blood from a newborn human to assay at least one analyte therefrom, comprising: (a) providing a sample of blood from a newborn human; (b) placing aliquots of the blood in a plurality of cells of a test tray, the test tray comprising: (i) a plurality of cells; and (ii) at least two of the cells containing a dried internal standard (as described above) used in each of the tests; so as to produce a respective plurality of test/standard samples; and (c) performing an assay test on each of the respective plurality of test/standard samples.

The blood spot extracts, containing the isotopically labelled standards may then be subject to chemical modification or analyzed directly. All conditions to which the sample is exposed also apply to the internal standard.

The tray may be made of any appropriate material, such as plastic or metal or combinations, and may contain cells of varying size and number. The cells may number preferably from 4 to 96. These may be arrayed in accordance with the number of tests to be conducted. The cells typically may be one-fourth inch to three-fourths inch in diameter.

The bodily fluid that may be tested is any bodily fluid upon which tests are performed and may include blood, semen, saliva, urine or spinal fluid.

The standards may be dried according to known methods including heat drying and freeze drying (lyophilization).

The number of tests that may be performed with each test tray may vary with the type of standard deposited in each cell. The tray may have all cells with a single standard or may contain one or more series adapted to perform one of a series of tests; for example, twenty cells with five series of four standards for four iterations of five different tests.

The preferred embodiment of the present invention utilizes dried isotopically labelled standards for newborn screening which are dissolved and used on a sample-by-sample basis. This avoids loss of solvent and consequent changes in internal standard concentration.

Typically, blood spots from newborns are taken from paper sample cards by punching portions of the blood spots to create samples. This is done using punching devices known in the art. The design of sampling cards varies, but typically the sampling cards contain from b 2 to 10 spots per card.

The spot punches are placed in the individual to cells in a multi-cell test tray. The blood samples are typically solvated, such as through the use of solvent such as methanol or ethanol.

At this point, the solvated samples are then transferred to a second multi-cell test tray. The sample that is typically derivatized to prepare the sample so that the molecules (i.e. typically amino acids and fatty acids) will be presented to the mass spectrometer in a protonated form. Typically, a derivatizing agent such as butanol-n-HCI is used for this purpose, or other appropriate derivatizing agent.

Transference of samples may be done in accordance with methods and through use of equipment known and used in the art, such as through use of multi-channel micropipettes and robotic dispensers.

The samples must then be dried again to remove excess derivatizing agent.

Finally, a protonation agent, such as a combination of acetonitrile and water, is added prior to the sample being analyzed by tandem mass spectroscopy. An internal standard, typically reconstituted from a dried the state into a liquid, is the added prior to the sample being analyzed.

In accordance with a preferred embodiment of the invention, the blood spot punches are placed in a multi-cell test tray, the test tray having cells already provided with a dried internal standard.

The blood samples and the dried internal standards in each cell of the test tray are solvated by addition of a protonation agent/solvent, such as a mixture of methanol and water.

In the preferred embodiment of the invention, the solvated test sample/standards are directly introduced into a tandem mass spectrometer that is adapted to process the test sample/standards using an electrospray. The tandem mass spectrometer may be any appropriate spectrometer such as, for instance, those commercially available from MDS SCIEX of Concord, Ontario Canada.

The preferred embodiment the invention thus eliminates the need to transfer, derivative and dry the test sample/standard mixtures from each cell. The preferred embodiment invention also eliminates the use of a separately prepared or stored standard solution, as the internal standard is reconstituted along with the spotted blood sample. This is also advantageous because stored internal standards are subject to contamination or changes in concentration owing to evaporation of the solvent (typically an alcohol). The preferred embodiment of the present invention overcomes the change in standard concentration by depositing solid samples of the standards in individual microtiter plate wells. These standards are then dissolved as the blood spot is extracted, and used in the same procedure. Any evaporation of the standard solvent and consequent uncertainty or error in the calculation of the amount of analyte present is avoided by treating the standard exactly the same as the analyte from the extraction step on through the procedure. Changes of concentration of the standard by evaporation are avoided by storing them dry, with no opportunity for the solvent to evaporate prior to use. Degradation of the acylcarnitine standards by hydrolysis is avoided by sealing the plate under dry air, followed by sealing the sealed plate in a desiccated package.

The methods for newborn screening may use testing protocols such as those described in Rashed et al., "Diagnosis of Inborn Errors of Metabolism from Blood Spots by Acylcarnitines and Amino Acids Profiling Using Automated Electrospray Tandem Mass Spectroscopy," Pediatric Research, Vol. 38, no. 3, pp. 324-331 (1995); Chace et al., "Rapid Diagnosis of Homocystinuria and other Hypermethionimias from Newborns' Blood Spots by Tandem Mass Spectroscopy," Clinical Chemistry, Vol. 42:3, pp. 349-355 (1996); Millington, et al., "Tandem Mass Spectroscopy: A New Method for Acylcarnitine Profiling with Potential for Neonatal Screening for Inborn Errors of Metabolism," J. Inher. Metab. Dis. Vol. 13, pp. 321-324 (1990); Rashed et al., "Screening Blood Spots for Inborn Errors of Metabolism by Electrospray Tandem Mass Spectroscopy with a Microplate Bach Process and a Computer Algorithm for Automated Flagging," Clinical Chemistry, Vol. 43:7, pp. 1129-1141 (1997); Magera et al., "Method for the Determination of Total Homocysteine in Plasma and Urine by Stable Isotope Dilution and Electrospray Tandem Mass Spectroscopy," Clinical Chemistry, Vol. 45:9, pp. 1517-1522 (1999); Chace et al., "Use of Phenylalanine-to-Tyrosine Ratio Determined by Tandem Mass Spectrometry to Improve Newborn Screen for Phenylketonuria of Early Discharge Specimens Collected in the First 24 Hours," Clinical Chemistry, Vol. 44:12, pp. 2405-2409 (1998); Chace et al., "Rapid Diagnosis of Phenylketonuria by Quantitative Analysis for Phenylalanine and Tyrosine in Neonatal Blood Spots by Tandem Mass Spectrometry," Clinical Chemistry, Vol. 39:1, pp. 66-71 (1993); Chace et al., "Rapid Diagnosis of MCAD Deficiency: Quantitative Analysis of Octanoylcarnitine and Other Acylcarnitines in Newborn Blood Spots by Tandem Mass Spectrometry," Clinical Chemistry, Vol. 43:11, pp. 2106-2113 (1997); Chace, et al., "Rapid Diagnosis of Phenylketonuria by Quantitative Analysis for Phenylalanine and Tyrosine in Neonatal Blood Spots by Tandem Mass Spectrometry," Clinical Chem. vol. 38:66-71 (1993); Chace, et al., "Rapid Diagnosis of Maple Syrup Urine Disease in Blood Spots from Newborns by Tandem Mass Spectrometry," Clinical Chem. vol. 41:62-8 (1995); Rashed, et al., "Electrospray Tandem Mass Spectrometry in the Diagnosis of Organic Acidemias"' Rapid Commun. Mass Spectrom. 8:129-33 (1994); Rashed, et al., "Diagnosis of Inborn Errors of Metabolism from Blood Spots by Acylcarnitines and Amino Acid Profiling using Automated Electrospray Tandem Mass Spectrometry," Pediatric Res. 38:324-331 (1995); Rinaldo, et al. "Medium Chain Acyl-CoA Dehydrogenase Deficiency. Diagnosis by Stable Isotope Dilution Measurement of Urinary N-Hexanoylglycine and 3-Phenylpropionylglycine," New Eng. J. Med. Vol. 319:1308-1313 (1988); Magera, et al. "Method for the Determination of Total Homocysteine in Plasma and Urine by Stable Isotope Dilution and Electrospray Tandem Mass Spectrometry," Clinical Chemistry, vol. 45:1517-22 (1999); Chace et al., "Rapid Diagnosis of Homocystinuria and Other Hypermethioninemias from Newborns' Blood Spots by Tandem Mass Spectrometry," Clinical Chem. Vol. 42:349-355 (1996); Rashed, et al. "Screening Blood Spots for Inborn Errors of Metabolism by Electrospray Tandem Mass Spectrometry with a Microplate Batch Process and a Computer Algorithm for Automated Flagging of Abnormal Profiles. Clinical Chem., vol. 43:1129-42 (1997); Lowes et al., "Identification of Urinary Acylcarnitines using Gas Chromatography-Mass Spectrometry: Preliminary Clinical Applications," J. Chromatogr. Vol. 577:205-14 (1992); Millington et al., "Tandem Mass Spectrometry: A New Method for Acylcarnitine Profiling for Neonatal Screening for Inborn Errors of Metabolism," Journal of Inher. Metab. Dis. Vol. 13:321-324 (1990); Millington et al., "The Analysis of Diagnostic Markers of Genetic Disorders in Human Blood and Urine using Tandem Mass Spectrometry with Liquid Secondary Ion Mass Spectrometry. Int. J. Mass Spectrom. Ion Processes," vol. 111: pp. 211-28 (1991); Millington et al., "New Developments in Fatty Acid Oxidation. In: Coates P M, Tanaka K eds. The Role of Tandem Mass Spectrometry in the Diagnosis of Fatty Acid Oxidation Disorders," New York: Wiley-Liss, pp. 339-54 (1992); Chace, et al., "Rapid Diagnosis of MCAD Deficiency: Quantitative Analysis of Octanoylcarnitine and Other Acylcarnitines in Newborn Blood Spots by Tandem Mass Spectrometry," Clinical Chemistry, vol. 43:11, pp. 2106-2113 (1997); Chace, et al., "Use of Phenylalanine-to-Tyrosine Ratio Determined by Tandem Mass Spectrometry to Improve Newborn Screening for Phenylketonuria of Early Discharge Specimens Collected in the First 24 Hours," Clinical Chemistry, vol. 44:12, pp. 2405-2409 (1998); and Rashed, et al. "Diagnosis of Inborn Errors of Metabolism from Blood Spots by Acylcarnitines and Amino Acids Profiling Using Automated Electrospray Tandem Mass Spectrometry," Clinical Chemistry, Vol. 38, No. 3 (1998).

Also included in the present invention is a method of making a test tray comprising a plurality of test cells for conducting a plurality of tests on a biological fluid, the method comprising: (a) placing an aliquot of a liquid containing an internal standard as described above in each of the test cells of the tray; and (b) drying the liquid containing an internal standard (as described herein), so as to form a layer of a pre-determined amount of dried internal standard upon the surface of each cell in the tray.

Drying may be carried out by any method appropriate to treatment of the internal standard, such as heat drying or lyophilization.

### DETAILED DESCRIPTION OF THE PREFERRED EMBODIMENT

As described above.

In light of the foregoing disclosure, it will be within the ability of one skilled in the newborn screening art to make modifications to the present invention, such as through the substitution of equivalent chemicals, compounds and their concentrations, or the application of equivalent process steps.

## Claims

1. A test tray for conducting a plurality of tests on a biological fluid; said tray comprising:
(a) a plurality of cells; and
(b) at least two of said cells containing a dried internal isotope labeled standard selected from the group consisting of carnitine, acetyl carnitine, propionyl carnitine, butryl carnitine, isovaleryl carnitine, octanoyl carnitine, myristoyl carnitine, palmitoyl carnitine, glycine, alanine, valine, leucine, methionine, phenylalanine, tyrosine, aspartate, glutamate, ornithine, citrulline, arginine, polysaccharides, polysaccharides bound to protein or protein fragments, and mixtures thereof.

2. The test tray according to claim 1 additionally comprising a cover piece disposed over said cells.

3. A method of conducting a plurality of in vitro tests to assay analytes in a bodily fluid sample, the method comprising the steps of:
(a) providing a sample of bodily fluid
(b) placing aliquots of said bodily fluid sample in a plurality of cells of a test tray, said test tray comprising:
(i) a plurality of cells;
(ii) at least two of said cells containing a dried internal isotope labeled standard selected from the group consisting of carnitine, acetyl carnitine, propionyl carnitine, butryl carnitine, isovaleryl carnitine, octanoyl carnitine, myristoyl carnitine, palmitoyl carnitine, glycine, alanine, valine, leucine, methionine, phenylalanine, tyrosine, aspartate, glutamate, ornithine, citrulline, arginine, polysaccharides, polysaccharides bound to protein or protein fragments, and mixtures thereof used in each of said tests; so as to produce a respective plurality of test/standard samples; and
(c) performing an assay test on each of said respective plurality of test/standard samples.

4. The method of conducting a plurality of tests according to claim 3, wherein said tests performed on each of said respective plurality of test/standard samples includes at least two tests adapted to assay different analytes from respective of said plurality of test/standard samples.

5. The method of conducting a plurality of tests according to claim 3, wherein said test includes at least two tests adapted to assay the same analyte from said respective plurality of test/standard samples.

6. The method of conducting a plurality of tests according to claim 3, wherein said test includes tandem mass spectrometry.

7. A kit for conducting a plurality of tests to assay analytes in a bodily fluid, said kit comprising:
(a) the test tray of claim 1; and
(b) a solvent for addition to said cells containing a dried internal standard.

8. The kit according to claim 7 additionally comprising a solvent dispenser.

9. A method of conducting a plurality of tests on blood from a newborn human to assay at least one analyte therefrom, said method comprising:
(a) obtaining a test tray, said test tray comprising:
(i) a plurality of cells; and
(ii) at least two of said cells containing a dried internal standard used in each of said tests wherein said dried internal standard is an isotope labeled standard selected from the group consisting of carnitine, acetyl carnitine, propionyl carnitine, butryl carnitine, isovaleryl carnitine, octanoyl carnitine, myristoyl carnitine, palmitoyl carnitine, glycine, alanine, valine, leucine, methionine, phenylalanine, tyrosine, aspartate, glutamate, ornithine, citrulline, arginine, polysaccharides, polysaccharides bound to protein or protein fragments, and mixtures thereof;
(b) placing a plurality of sample paper portions each bearing dried sample of blood from a newborn human into respective cells of said test tray;
(c) placing aliquots of said blood into respective cells of said test tray so as to produce a respective plurality of test/standard samples; and
(d) performing tandem mass spectrometry on each of said respective plurality of test/standard samples.

10. The method of conducting a plurality of tests according to claim 9, wherein said tests performed on each of said respective plurality of test/standard samples includes at least two tests adapted to assay different analytes from respective of said plurality of test/standard samples.

11. The method of conducting a plurality of tests according to claim 9, wherein said test includes at least two tests adapted to assay the same analyte from said respective plurality of test/standard samples.

12. A method of conducting a plurality of in vitro tests to assay analytes in a blood sample, the method comprising the steps of:
(a) providing a sample of blood from a newborn human;
(b) placing aliquots of said blood in a plurality of cells of a test tray, said test tray comprising:
(i) a plurality of cells; and
(ii) at least two of said cells containing a dried internal standard used in each of said tests wherein said dried internal standard is an isotope labeled standard selected from the group consisting of carnitine, acetyl carnitine, propionyl carnitine, butryl carnitine, isovaleryl carnitine, octanoyl carnitine, myristoyl carnitine, palmitoyl carnitine, glycine, alanine, valine, leucine, methionine, phenylalanine, tyrosine, aspartate, glutamate, ornithine, citrulline, arginine, polysaccharides, polysaccharides bound to protein or protein fragments, and mixtures thereof; so as to produce a respective plurality of test/standard samples; and
(c) performing an assay test on each of said respective plurality of test/standard samples.

13. A method of making a test tray comprising a plurality of test cells for conducting a plurality of tests on a biological fluid; said method comprising:
(a) placing an aliquot of a liquid containing an internal standard is each of said test cells of said tray; and
(b) drying said liquid containing an internal standard, so as to form a layer of dried internal standard upon the surface of each cell in said tray; wherein said dried internal standard is an isotope labeled standard selected from the group consisting of carnitine, acetyl carnitine, propionyl carnitine, butryl carnitine, isovaleryl carnitine, octanoyl carnitine, myristoyl carnitine, palmitoyl carnitine, glycine, alanine, valine, leucine, methionine, phenylalanine, tyrosine, aspartate, glutamate, ornithine, citrulline, arginine, polysaccharides, polysaccharides bound to protein or protein fragments, and mixtures thereof.

14. The method of making a test tray according to claim 13, wherein said drying is done by lyophilization.

## Patentansprüche

1. Testschale zur Durchführung einer Vielzahl von Tests an einer biologischen Flüssigkeit;
wobei die Schale umfasst:
(a) eine Vielzahl von Zellen; und
(b) wobei mindestens zwei der Zellen einen getrockneten internen isotopenmarkierten Standard enthalten, ausgewählt aus der Gruppe, bestehend aus Carnitin, Acetylcarnitin, Propionylcarnitin, Butyrylcarnitin, Isovalerylcarnitin, Octanoylcarnitin, Myristoylcarnitin, Palmitoylcarnitin, Glycin, Alanin, Valin, Leucin, Methionin, Phenylalanin, Tyrosin, Aspartat, Glutamat, Ornithin, Citrullin, Arginin, Polysacchariden, protein- oder proteinfragment-gebundenden Polysacchariden und Mischungen davon.

2. Testschale gemäß Anspruch 1, zusätzlich umfassend ein Abdeckteil, das über den Zellen angeordnet ist.

3. Verfahren zur Durchführung einer Vielzahl von in-vitro-Tests, um Analyten in einer Körperflüssigkeitsprobe zu untersuchen, wobei das Verfahren die Schritte umfasst:
(a) Bereitstellen einer Probe einer Körperflüssigkeit
(b) Anordnen von Aliquots der Körperflüssigkeitsprobe in eine Vielzahl von Zellen einer Testschale, wobei die Testschale umfasst:
(i) eine Vielzahl von Zellen;
(ii) wobei mindestens zwei der Zellen einen getrockneten internen isotopenmarkierten Standard enthalten, ausgewählt aus der Gruppe, bestehend aus Carnitin, Acetylcarnitin, Propionylcarnitin, Butyrylcarnitin, Isovalerylcarnitin, Octanoylcarnitin, Myristoylcarnitin, Palmitoylcarnitin, Glycin, Alanin, Valin, Leucin, Methionin, Phenylalanin, Tyrosin, Aspartat, Glutamat, Ornithin, Citrullin, Arginin, Polysacchariden, protein- oder proteinfragment-gebundenden Polysacchariden und Mischungen davon, der in jedem der Tests verwendet wird; um eine entsprechende Vielzahl an Test-/Standardproben zu erzeugen; und
(c) Durchführung eines Untersuchungstests bei jeder der entsprechenden Vielzahl an Test-/Standardproben.

4. Verfahren zur Durchführung einer Vielzahl von Tests gemäß Anspruch 3, wobei die Tests, die bei jeder der entsprechenden Vielzahl an Test-/Standardproben durchgeführt werden, mindestens zwei Tests einschließen, die angepasst sind, um unterschiedliche Analyten von entsprechenden der Vielzahl an Test-/Standardproben zu untersuchen.

5. Verfahren zur Durchführung einer Vielzahl von Tests gemäß Anspruch 3, wobei der Test mindestens zwei Tests einschließt, die angepasst sind, um den gleichen Analyten von der jeweiligen Vielzahl an Test-/Standardproben zu untersuchen.

6. Verfahren zur Durchführung einer Vielzahl von Tests gemäß Anspruch 3, wobei der Test Tandem-Massenspektrometrie einschließt.

7. Kit zur Durchführung eine Vielzahl von Tests, um Analyten in einer Körperflüssigkeit zu untersuchen, wobei das Kit umfasst:
(a) die Testschale aus Anspruch 1; und
(b) ein Lösungsmittel zur Zugabe zu den Zellen, das einen getrockneten internen Standard enthält.

8. Kit gemäß Anspruch 7, zusätzlich umfassend einen Lösungsmittelspender.

9. Verfahren zur Durchführung einer Vielzahl von Tests an Blut eines neugeborenen Menschen, um mindestens einen Analyten daraus zu untersuchen, wobei das Verfahren umfasst:
(a) Erhalten einer Testschale, wobei die Testschale umfasst:
(i) eine Vielzahl von Zellen; und
(ii) wobei mindestens zwei der Zellen einen getrockneten internen Standard enthalten, der bei jedem der Tests verwendet wird, wobei der getrocknete interne Standard ein isotopenmarkierter Standard ist, ausgewählt aus der Gruppe, bestehend aus Carnitin, Acetylcarnitin, Propionylcarnitin, Butyrylcarnitin, Isovalerylcarnitin, Octanoylcarnitin, Myristoylcarnitin, Palmitoylcarnitin, Glycin, Alanin, Valin, Leucin, Methionin, Phenylalanin, Tyrosin, Aspartat, Glutamat, Ornithin, Citrullin, Arginin, Polysacchariden, protein- oder proteinfragment-gebundenden Polysacchariden und Mischungen davon;
(b) Anordnen einer Vielzahl von Probenpapierabschnitten, die jeder getrocknete Blutproben eines neugeborenen Menschen tragen, in die jeweiligen Zellen der Testschale;
(c) Anordnen von Aliquots des Blut in die jeweiligen Zellen der Testschale, um eine entsprechende Vielzahl an Test-/Standardproben zu erzeugen; und
(d) Durchführen einer Tandem-Massenspektrometrie bei jeder der jeweiligen Vielzahl an Test-/Standardproben.

10. Verfahren zur Durchführung einer Vielzahl von Tests gemäß Anspruch 9, wobei die Tests, die bei jeder der jeweiligen Vielzahl an Test-/Standardproben durchgeführt werden, mindestens zwei Tests einschließen, die angepasst sind, um unterschiedliche Analyten vom jeweiligen der Vielzahl an Test-/Standardproben zu untersuchen.

11. Verfahren zur Durchführung einer Vielzahl von Tests gemäß Anspruch 9, wobei der Test mindestens zwei Tests einschließt, die angepasst sind, um den gleichen Analyten von der entsprechenden Vielzahl an Test-/Standardproben zu untersuchen.

12. Verfahren zur Durchführung einer Vielzahl von in-vitro-Tests, um Analyten in einer Blutprobe zu untersuchen, wobei das Verfahren die Schritte umfasst:
(a) Bereitstellen einer Blutprobe eines neugeborenen Menschen;
(b) Anordnen von Aliquots des Bluts in eine Vielzahl von Zellen einer Testschale, wobei die Testschale umfasst:
(i) eine Vielzahl von Zellen; und
(ii) wobei mindestens zwei der Zellen einen getrockneten internen Standard enthalten, der in jedem der Tests verwendet wird, wobei der getrocknete interne Standard ein isotopenmarkierter Standard ist, ausgewählt aus der Gruppe, bestehend aus Carnitin, Acetylcarnitin, Propionylcarnitin, Butyrylcarnitin, Isovalerylcarnitin, Octanoylcarnitin, Myristoylcarnitin, Palmitoylcarnitin, Glycin, Alanin, Valin, Leucin, Methionin, Phenylalanin, Tyrosin, Aspartat, Glutamat, Ornithin, Citrullin, Arginin, Polysacchariden, protein- oder proteinfragment-gebundenden Polysacchariden und Mischungen davon; um eine entsprechende Vielzahl an Test-/Standardproben zu erzeugen; und
(c) Durchführung eines Untersuchungstests bei jeder der jeweiligen Vielzahl an Test-/Standardproben.

13. Verfahren zur Herstellung einer Testschale, die eine Vielzahl von Test-Zellen umfasst, zur Durchführung einer Vielzahl von Tests an einer biologischen Flüssigkeit; wobei das Verfahren umfasst:
(a) Anordnen eines Aliquots einer Flüssigkeit, die einen internen Standard enthält, in jede der Test-Zellen der Schale; und
(b) Trocknen der Flüssigkeit, die einen internen Standard enthält, um eine Schicht aus getrocknetem internem Standard auf der Oberfläche jeder Zelle in der Schale zu bilden; wobei der getrocknete interne Standard ein isotopenmarkierter Standard ist, ausgewählt aus der Gruppe, bestehend aus Carnitin, Acetylcarnitin, Propionylcarnitin, Butyrylcarnitin, Isovalerylcarnitin, Octanoylcarnitin, Myristoylcarnitin, Palmitoylcarnitin, Glycin, Alanin, Valin, Leucin, Methionin, Phenylalanin, Tyrosin, Aspartat, Glutamat, Ornithin, Citrullin, Arginin, Polysacchariden, protein- oder proteinfragment-gebundenen Polysacchariden und Mischungen davon.

14. Verfahren zur Herstellung einer Testschale gemäß Anspruch 13, wobei das Trocknen durch Gefriertrocknung vorgenommen wird.

## Revendications

1. Cuvette d'essai pour réaliser une pluralité de tests sur un fluide biologique ; ladite cuvette comprenant :
(a) une pluralité de cellules ; et
(b) au moins deux desdites cellules contenant un standard interne sec isotopiquement marqué choisi dans le groupe consistant en carnitine, acétyl carnitine, propionyl carnitine, butyryl carnitine, isovaléryl carnitine, octanoyl carnitine, myristoyl carnitine, palmitoyl carnitine, glycine, alanine, valine, leucine, méthionine, phénylalanine, tyrosine, aspartate, glutamate, ornithine, citrulline, arginine, polysaccharides, polysaccharides liés à des protéines ou à des fragments de protéines, et leurs mélanges.

2. Cuvette d'essai selon la revendication 1 comprenant de plus un élément de couvercle disposé sur lesdites cellules.

3. Procédé de réalisation d'une pluralité de tests in vitro pour essayer des analytes dans un échantillon de fluide corporel, le procédé comprenant les étapes consistant à :
(a) procurer un échantillon de fluide corporel
(b) placer des aliquotes dudit échantillon de fluide corporel dans une pluralité de cellules d'une cuvette d'essai, ladite cuvette d'essai comprenant :
(i) une pluralité de cellules ;
(ii) au moins deux desdites cellules contenant un standard interne sec isotopiquement marqué choisi dans le groupe consistant en carnitine, acétyl camitine, propionyl carnitine, butyryl carnitine, isovaléryl carnitine, octanoyl carnitine, myristoyl carnitine, palmitoyl carnitine, glycine, alanine, valine, leucine, méthionine, phénylalanine, tyrosine, aspartate, glutamate, ornithine, citrulline, arginine, polysaccharides, polysaccharides liés à des protéines ou à des fragments de protéines, et leurs mélanges, utilisés dans chacun desdits tests ; de manière à produire une pluralité respective d'échantillons de tests/standards ; et
(c) réaliser un test d'essai de chacun de ladite pluralité respective d'échantillons de tests/standards.

4. Procédé de réalisation d'une pluralité de tests selon la revendication 3, dans lequel lesdits tests réalisés sur chacun de ladite pluralité respective d'échantillons de tests/standards comprennent au moins deux tests adaptés pour essayer divers analytes de ladite pluralité respective d'échantillons de tests/standards.

5. Procédé de réalisation d'une pluralité de tests selon la revendication 3, dans lequel ledit test inclut au moins deux tests adaptés pour essayer le même analyte de ladite pluralité respective d'échantillons de tests/standards.

6. Procédé de réalisation d'une pluralité de tests selon la revendication 3, dans lequel ledit test inclut la spectrométrie de masse en tandem.

7. Trousse pour réaliser une pluralité de tests pour essayer des analytes dans un fluide corporel, ladite trousse comprenant :
(a) la cuvette d'essai de la revendication 1 ; et
(b) un solvant pour l'addition aux dites cellules contenant un standard interne sec.

8. Trousse selon la revendication 7 comprenant en outre un distributeur de solvant.

9. Procédé de mise en oeuvre d'une pluralité de tests sur du sang d'un nouveau-né humain pour en essayer au moins un analyte, ledit procédé consistant à :
(a) obtenir une cuvette d'essai, ladite cuvette d'essai comprenant :
(i) une pluralité de cellules ; et
(ii) au moins deux desdites cellules contenant un standard interne sec utilisé dans chacun desdits tests où ledit standard interne est un standard marqué avec un isotope sélectionné dans le groupe consistant en carnitine, acétyl carnitine, propionyl carnitine, butyryl carnitine, isovaléryl carnitine, octanoyl carnitine, myristoyl carnitine, palmitoyl carnitine, glycine, alanine, valine, leucine, méthionine, phénylalanine, tyrosine, aspartate, glutamate, ornithine, citrulline, arginine, polysaccharides, polysaccharides liés à des protéines ou à des fragments de protéines, et leurs mélanges ;
(b) placer une pluralité de morceaux de papier comportant chacun un échantillon séché de sang d'un nouveau-né humain dans les cellules respectives de ladite cuvette d'essai ;
(c) placer des aliquotes dudit sang dans les cellules respectives de ladite cuvette d'essai de manière à produire une pluralité respective d'échantillons d'essais/standards ; et
(d) effectuer une spectrométrie de masse en tandem sur chacun de ladite pluralité respective d'échantillons de tests/standards.

10. Procédé de mise en oeuvre d'une pluralité de tests selon la revendication 9, dans lequel lesdits tests effectués sur chaque pluralité respective d'échantillons de tests/standards inclut au moins deux tests adaptés pour essayer différents analytes de la pluralité respective d'échantillons de tests/standards.

11. Procédé de mise en oeuvre d'une pluralité de tests selon la revendication 9, dans lequel ledit test inclut au moins deux tests adaptés pour essayer le même analyte de ladite pluralité respective d'échantillons de tests/standards.

12. Procédé de mise en oeuvre d'une pluralité de tests in vitro pour essayer des analytes dans un échantillon de sanguin, le procédé comprenant les étapes consistant à :
(a) obtenir un échantillon de sang d'un nouveau-né humain ;
(b) placer des aliquotes dudit sang dans une pluralité de cellules d'une cuvette d'essai, ladite cuvette d'essai comprenant :
(i) une pluralité de cellules ; et
(ii) au moins deux desdites cellules contenant un standard interne sec utilisé dans chacun desdits tests où ledit standard interne est un standard marqué isotopiquement sélectionné parmi le groupe consistant en carnitine, acétyl camitine, propionyl carnitine, butyryl carnitine, isovaléryl carnitine, octanoyl carnitine, myristoyl carnitine, palmitoyl carnitine, glycine, alanine, valine, leucine, méthionine, phénylalanine, tyrosine, aspartate, glutamate, ornithine, citrulline, arginine, polysaccharides, polysaccharides liés à des protéines ou à des fragments de protéines, et leurs mélanges ; de manière à produire une pluralité respective d'échantillons de tests/standards ; et
(c) réaliser un test d'essai sur chacun de ladite pluralité respective d'échantillons de tests/standards.

13. Procédé de fabrication d'une cuvette de test comprenant une pluralité de cellules de test pour effectuer une pluralité de tests sur un fluide biologique ; ledit procédé consistant à :
(a) placer une aliquote d'un liquide contenant un standard interne dans chacune des cellules de test de ladite cuvette ; et
(b) sécher ledit liquide contenant un standard interne, de manière à former une couche de standard interne séché sur la surface de chaque cellule dans ladite cuvette ; dans lequel ledit standard interne sec est un standard isotopiquement marqué choisi dans le groupe consistant en carnitine, acétyl carnitine, propionyl carnitine, butyryl carnitine, isovaléryl carnitine, octanoyl carnitine, myristoyl carnitine, palmitoyl carnitine, glycine, alanine, valine, leucine, méthionine, phénylalanine, tyrosine, aspartate, glutamate, ornithine, citrulline, arginine, polysaccharides, polysaccharides liés à des protéines ou à des fragments de protéines, et leurs mélanges.

14. Procédé de réalisation d'une cuvette de test selon la revendication 13, dans lequel ledit séchage est effectué par lyophilisation.
